# Europäisches Patentamt
# European Patent Office
# Office européen des brevets

(11) Publication number: **0 059 182**
**B1**

(12) **EUROPEAN PATENT SPECIFICATION**

(45) Date of publication of patent specification: **21.11.84**

(21) Application number: **81900726.1**

(22) Date of filing: **02.03.81**

(86) International application number:
**PCT/HU81/00008**

(87) International publication number:
**WO 82/00253 04.02.82 Gazette 82/04**

(51) Int. Cl.³: **A 61 K 35/23,** C 12 N 11/00,
C 12 N 9/00

(54) **A PROCESS FOR ISOLATING AMINOACYLASE ENZYME FROM A MAMMAL KIDNEY EXTRACT.**

(30) Priority: **17.07.80 HU 178280**

(43) Date of publication of application:
**08.09.82 Bulletin 82/36**

(45) Publication of the grant of the patent:
**21.11.84 Bulletin 84/47**

(84) Designated Contracting States:
**AT CH DE FR GB LI LU NL SE**

(56) References cited:
FR-A-2 198 954
GB-A-1 257 263
GB-A-1 289 549
US-A-2 769 749
US-A-3 616 231
US-A-3 794 563
US-A-3 933 589

BIOLOGICAL ABSTRACTS, vol. 73, 1982, ref.
53185, COLUMBUS OHIO (US)
BIOLOGICAL ABSTRACTS, vol. 71, 1981, ref.
80277, COLUMBUS OHIO (US)
CHEMICAL ABSTRACTS, vol. 93, no. 21,
November 24, 1980, page 295, ref. 200119c,
COLUMBUS OHIO (US)
BIOLOGICAL ABSTRACTS, vol. 70, 1980, ref.
77842, COLUMBUS OHIO (US)

(73) Proprietor: **REANAL FINOMVEGYSZERGYAR**
**P.O. Box 54 Telepes u. 53**
**H-1441 Budapest (HU)**

(72) Inventor: **BOROS, László**
**Pauler u. 4**
**H-1013 Budapest (HU)**
Inventor: **DAROCZI, Iván**
**Dessewffy u. 28**
**H-1066 Budapest (HU)**
Inventor: **HUBER, Irén**
**Villányi u. 86**
**H-1118 Budapest (HU)**
Inventor: **IVONY, Józsefné**
**Mezö, Imre u. 21-23**
**H-1081 Budapest (HU)**
Inventor: **KISS, Jánosné**
**Vajda u. 36/b**
**H-6723 Szeged (HU)**
Inventor: **SZAJANI, Béla**
**Tusnádi u. 26**
**H-1125 Budapest (HU)**

(74) Representative: **Beszédes, Stephan G. Dr.**
**Münchener Strasse 80a Postfach 1168**
**D-8060 Dachau (DE)**

(56) References cited:
**BIOLOGICAL ABSTRACTS, vol. 73, 1982, ref.**
**53184, COLUMBUS OHIO (US)**

Courier Press, Leamington Spa, England.

## Description

The invention relates to an improved method for isolating aminoacylase enzyme from a mammal kidney extract, particularly pig kidney extract.

It is known that aminoacylase enzyme occurs in the highest concentration in pig kidneys, and therefore in the known methods for isolating aminoacylase enzyme pig kidneys are utilized as a raw material.

According to the method described in J. Biol. Chem. *178*, 503 (1949) aminoacylase is isolated from pig kidney cortex as follows: The aqueous extract of the pig kidney cortex is admixed with 78% ethanol to attain an ethanol concentration of 15%, the mixture is allowed to stand at −6°C for 12 hours, then centrifuged, and the ethanol content of the supernatant is adjusted to 30%. The pH of the resulting liquid is adjusted to 6.3, and the mixture is allowed to stand at −15°C for further 12 hours. The mixture is centrifuged to remove the precipitate, the pH of the supernatant is adjusted to 5.1, and the mixture is again allowed to stand for 12 hours. The separated precipitate, which contains the aminoacylase enzyme, is isolated by centrifuging. The specific activity of the resulting substance is about fourfold of that of the starting extract, which corresponds to a very low degree of purification.

In J. Biol. Chem. *194*, 455 (1952) a series of operations consisting of six fractionation steps and a subsequent freeze-drying step is disclosed for the isolation of aminoacylase from an aqueous extract of pig kidneys. This method yields the required enzyme with an about 30-fold (i.e. still relatively low) purification grade, and it is much more laboursome than the method discussed above. The specific activity of the freeze-dried product is only 4,640 units/mg.

Aminoacylase can be isolated in very pure state, with a specific activity of 15,686 units/mg, from an aqueous extract of pig kidneys by the method disclosed in Biochem. Z. *336*, 162 (1962). The disadvantage of this method is, however, that it consists of nine lengthy, time-consuming and laboursome separation and purification steps.

According to the method disclosed in Biochimiya *22*, 838 (1957) an acetone powder made of pig kidney is applied as a starting substance. The acetone powder is extracted with a phosphate buffer, the extract is saturated with ammonium sulphate in two steps, and the resulting suspension is dialysed. The solution obtained after dialysis is subjected to heat treatment at 60°C for 5 minutes, then it is fractionated in two steps with saturated ammonium sulphate solution. The resulting precipitate is dissolved in water, the solution is dialysed again, and then it is fractionated in two steps with acetone. The precipitate obtained in the last fractionation step is dissolved in water, the solution is dialysed against distilled water, and then freeze-dried. By this method, consisting of 12 operation steps, a product with a specific activity of about 15,000 units/mg can be obtained.

As it appears from the above, a common disadvantage of the known methods is that they yield the required enzyme in a low or moderate purity, and the preparation of an appropriately pure product requires a complex series of operations, consisting of 9 to 12 purification steps.

Furthermore it has been known from US-Patent 3,616,231 a process of isolating uricase from animal tissue comprising the steps of extracting the water-insoluble fraction of an animal tissue homogenate with an alkaline salt solution, contacting the alkaline salt solution containing extracted uricase with kieselguhr whereby the uricase is adsorbed onto the kieselguhr, separating the kieselguhr having uricase adsorbed thereon and extracting this kieselguhr with alkaline acetone solution, treating the alkaline acetone extract with ammonium sulphate to precipitate the uricase present therein, and separating the thusly formed precipitate comprising uricase and extracting the same at least one time with a member selected from the group consisting of pure water and water containing a small amount of a salt to purify the same. In this process also a treatment of the uricase precipitate dissolved in the aqueous alkaline solution with solid calcium phosphate gel to remove any coloured impurities can be carried out.

The problem underlying to the invention is by eliminating the said disadvantages of the known methods to provide for a process for isolating aminoacylase enzyme from a mammal kidney extract which requires only few purification steps which are easy to perform, and yields the aminoacylase with a high specific activity.

The invention is based on the recognition that when an aqueous extract of a mammel kidney, particularly pig kidney, is subjected to heat treatment, then centrifuged, the supernatant is saturated with a salt of a monovalent cation formed with a mineral acid, the precipate is separated from the resulting suspension, dissolved in water and the solution is dialysed against water, and either the enzyme-containing solid is separated from the dialysed solution or the dialysed solution or a solution of the enzyme separated from the dialysate is subjected to the purifying steps as defined below and thereafter the enzyme is precipitated, an aminoacylase enzyme powder with a specific activity of about 3,000 to 3,600 units/mg is obtained. The enzyme separated from the dialysed solution can be utilized directly, without any subsequent purification, in the production of immobilized aminoacylase preparations. If, however, the enzyme powder obtained as described above is subjected to two chromatographic purification steps, a very pure aminoacylase product is obtained with a specific activity of about 15,000 units/mg. Thus this process, consisting of only three separation and two purification steps, yields the required aminoacylase enzyme in an extremely pure state. It has also been observed that this process enables one to isolate very pure aminoacylase with excellent results even from mammal kidneys different from pig kidney (such as horse, cattle or rabbit kidneys) which contain the enzyme in much lower concentrations and contaminated with much more ballast proteins than pig kidney.

Based on the above, the subject-matter of the invention is an improved process for isolating aminoacylase enzyme from a mammal kidney extract by means of a heat treatment, precipitating treatments with salts of the general formula $K_xA$ wherein K represents a monovalent cation, A is the anion of a mineral acid and x is equal to the valence of the anion, separating and dissolving precipitates in water and dialysis as well as optionally 1 or more purifying steps, which is characterized in that as a mammal kidney extract an aqueous kidney extract, prepared in a manner known per se, is subjected to heat treatment at 60 to 80°C for 5 to 15 minutes, the heat-treated mixture is centrifuged, 200 to 300 g/litre of a salt of the general formula $K_xA$ are added to the supernatant liquid, the resulting suspension is centrifuged, the separated precipitate is dissolved in water and the solution is dialysed against water and either

a) the enzyme-containing solid is separated from the dialysed solution in a manner known per se, or

b) $\alpha$) the dialysed solution or a solution of the enzyme separated from the dialysate in a manner known per se in a buffer of pH 6.5 to 8.5 is subjected to anion exchange, chromatography and, if desired,

$\beta$) the active fractions obtained in the chromatographic step or a solution of the enzyme precipitated from the active fractions with a salt of the general formula $K_xA$ in a buffer of pH 6.0 to 8.0 is subjected to gel chromatography on a gel filter wherein the upper limit of the fractionation molecular weight is higher than 70,000

and the enzyme is precipitated from the active fractions [obtained according to $\alpha$) or $\alpha$) and $\beta$)] with a salt of the general formula $K_xA$ as described above.

Between the process according to the invention and the process of US-Patent 3,616,231, there is already the fundamental difference that the former is concerned with isolating aminoacylase enzyme, whereas US-Patent 3,616,231 deals with the isolation of uricase which is not an enzyme of the aminoacylase type since it does not react with acylamino acids but with urea of an entirely different structure and specifically not in hydrolytic way but in oxidative way. Accordingly the process according to the invention is fundamentally different from the process of US-Patent 3,616,231 also regarding the process measures because according to the latter the water-insoluble fraction of an animal tissue homogenate is extracted with an alkaline salt solution, the extract containing the uricase is treated with kieselguhr, the kieselguhr having the uricase adsorbed thereon is separated from the remaining solution and the separated kieselguhr is eluted with an alkaline acetone solution which measures all are not carried out in the process according to the invention but rather just conversely it starts from an aqueous kidney extract which is heat-treated at a temperature from 60 to 80°C and the heat-treated mixture is centrifuged and only to the supernatant solution of this centrifuge gate is added a salt. Also, the dialysis of the process according to the invention has nothing to do with washing the precipitate with water according to US-Patent 3,616,231 since the former is carried out to free the respective solution from salts, whereas in the process according to US-Patent 3,616,231 the washing has the purpose to free the uricase of residual foreign proteins, that is it is a fractionation step. Likewise there is nothing common between the optional gel chromatography on a gel filter in the process according to the invention and the optional treatment with a calcium phosphate gel in the process according to US-Patent 3,616,231 since in the gel chromatography of the process according to the invention there is a fractionating separation according to the mass and the form of the molecules the mechanism of which is entirely different from the treatment with a calcium phosphate gel which is an adsorption chromatography for removing the coloured contaminations.

In the first step of the process according to the invention the aqueous kidney extract is heat-treated at 60 to 80°C for 5 to 15 minutes, preferably at 70°C for 10 minutes. This heat treatment denatures the majority of the proteins which accompany the enzyme to be isolated. The denaturated proteins separate from the aqueous solution as a precipitate with high surface area. This precipitate also acts as a purifier, and absorbs the majority of impurities remained in dissolved state. This explains why the purity grade obtained after six separation steps according to the method discussed in J. Biol. Chem. *194,* 455 (1952) can be well approached by the process of the invention already after three separation steps (heat treatment, precipitation, dialysis).

The aqueous solution obtained after the removal of the denaturated proteins is admixed with 200 to 300 g/l, preferably 250 to 280 g/l, particularly 260 to 270 g/l, of a salt of the general formula $K_xA$ to precipitate the crude enzyme. It is preferred to apply ammonium sulphate as precipitating agent of the general formula $K_xA$, however, other salts, such as ammonium chloride and sodium chloride, can be utilized as well. The separated precipitate is then dissolved in water, and the aqueous solution is dialysed against water in a manner known per se.

If desired, the enzyme-containing solid can be separated from the dialysed solution in a manner known per se, such as freeze-drying, spray-drying and evaporation. The specific activity of the resulting product, determined at 37°C, is about 3,000 to 3,600 units/mg which corresponds to a 20- to 25-fold purification efficiency. This product can be applied as such, without any further purification, for various industrial, purposes, e.g. to produce immobilized enzyme preparations.

If the crude enzyme obtained as described above is to be purified further, is it not absolutely necessary to separate the enzyme-containing solid from the dialysed solution. The dialysed solution can be applied directly, optionally after adjusting its pH, to an anion exchange column. It is preferred to apply Sephadex® resins, such as DEAE-Sephadex A-50®, as anion exchange resins. If a solid crude enzyme is applied as starting substance in the anion exchange chromatography, this solid is applied onto the anion exchange column as a solution in a buffer, such as in a 0.05 molar aqueous potassium phosphate buffer (pH=7.0). The column is equilibrated with the same buffer prior to applying the enzyme. The ion exchange column is eluted preferably with a 0.05 molar aqueous potassium phosphate buffer (pH=7.0) also containing sodium chloride, preferably 0.3 moles/litre of sodium chloride; however, other salt solutions with appropriate ionic strength can be applied as well for the same purpose.

The active fractions of the eluate are combined, and, if desired, the enzyme-containing solid is separated from the active fractions by the precipitation method discussed above. Generally 200 to 300 g, preferably 250 to 280 g, particularly 260 to 270 g, of a salt of the general formula $K_xA$ are added to one litre of the solution as precipitating agent. It is preferred to apply ammonium sulphate for this purpose. The specific activity of the separated solid, determined at 37°C is 6,000 to 6,600 units/mg which corresponds to a 40- to 45-fold purification efficiency in relation to the starting extract.

If the partially purified enzyme obtained as described above is to be purified further, it is not absolutely necessary to separate the enzyme-containing solid from the active fractions of the eluate. The active fractions can be applied directly, optionally after adjusting the pH, onto the gel column. It is preferred to apply Sephadex® gels, such as Sephadex G-200®, in the gel chromatographic purification. Only such gels can be applied, however, in this purification step for which the upper limit of the fractionation molecular weight range is higher than 70,000. If an enzyme-containing solid is applied as starting substance in gel chromatography, this solid is applied onto the gel column as a solution in a buffer, such as in a 0.05 molar aqueous potassium phosphate buffer (pH=7.0). The gel column is equilibrated with the same buffer prior to applying the enzyme. The gel column is eluted preferably with a 0.05 molar aqueous potassium phosphate buffer (pH=7.0), however, other dilute aqueous buffers can be applied as well. The active fractions of the effluent are combined, and then the enzyme is separated from the solution by the precipitation method discussed above. Generally 200 to 300 g, preferably 250 to 280 g, particularly 260 to 270 g, of a salt of the general formula $K_xA$ are added to one litre of the solution as precipitating agent. It is preferred to apply ammonium sulphate for this purpose. The specific activity of the separated enzyme, determined at 37°C, is about 15,000 units/mg; i.e. the purity grade of the product is excellent.

The crude, partially purified and further purified enzyme-containing solids obtained according to the process of the invention can be applied to advantage in the preparation of immobilized aminoacylase. As known, immobilized aminoacylase preparations are widely applied in the resolution of DL-amino acids.

The invention is elucidated in detail by the aid of the following non-limiting Examples.

Example 1
1 kg of purified pig kidney is comminuted and then homogenized with 2 litres of cold (+4°C) distilled water. The homogenizate is stirred for one hour on an ice water bath and then centrifuged. The supernatant is heated to 60°C, maintained at this temperature for 15 minutes, then cooled in an ice water bath and centrifuged. 280 g of ammonium sulphate are added to the supernatant, the mixture is allowed to stand overnight, and then the resulting suspension is centrifuged. The precipitate is dissolved in a small amount of cold (+4°C) distilled water, and the solution is dialyzed salt-free against distilled water. Thereafter the dialyzed solution is filtered and the filtrate is freeze-dried. 4.2 g of aminoacylase are obtained. Activity: hydrolysis of 1,000 $\mu$moles of N-acetyl-L-methionine/hour/mg of protein.

Example 2
1 kg of purified pig kidney is comminuted and then homogenized with 2 litres of cold (+4°C) distilled water. The homogenizate is stirred for one hour on an ice water bath and then centrifuged. The supernatant is heated to 80°C, maintained at this temperature for 5 minutes, then cooled in an ice water bath and centrifuged. 359 g of ammonium sulphate are added to the supernatant, the mixture is allowed to stand overnight, and the resulting suspension is centrifuged. The precipitate is dissolved in a small amount of cold (+4°C) distilled water, and the solution is dialyzed salt-free against distilled water. The dialyzed solution is filtered and the filtrate is freeze-dried. 1.1 g of aminoacylase are obtained. Activity: hydrolysis of 800 $\mu$moles of N-acetyl-L-methionine/hour/mg of protein.

Example 3

8 kg of purified pig kidney are comminuted and then homogenized with 16 liters of cold (+4°C) distilled water. The homogenizate is stirred for one hour on an ice water bath and then centrifuged. The supernatant is heated to 70°C, maintained at this temperature for 10 minutes, then cooled in an ice water bath and centrifuged. 2 554 g of ammonium sulphate are added to the supernatant, the mixture is allowed to stand overnight, and the resulting suspension is centrifuged. The precipitate is dissolved in a small amount of cold (+4°C) distilled water, and the solution is dialyzed salt-free against distilled water. The dialyzed solution is filtered, and the filtrate is freeze-dried. 7.2 g of aminoacylase are obtained. Activity: hydrolysis of 2,735 $\mu$moles of N-acetyl-L-methionine/hour/mg of protein.

Example 4

3.6 kg of purified cattle kidney are comminuted and then homogenized with 7.2 litres of cold (+4°C) distilled water. The homogenizate is stirred for one hour on an ice water bath and then centrifuged. The supernatant is heated to 70°C, maintained at this temperature for 10 minutes, then cooled in an ice water bath and centrifuged. 1,234 g of ammonium sulphate are added to the supernatant, the mixture is allowed to stand overnight, and the resulting suspension is centrifuged. The precipitate is dissolved in a small amount of cold (+4°C) distilled water, and the solution is dialyzed salt-free against distilled water. The dialyzed solution is filtered, and the filtrate is freeze-dried. 3.4 g of aminoacylase are obtained. Activity: hydrolysis of 791 $\mu$moles of N-acetyl-L-methionine/hour/mg of protein.

Example 5

2.5 kg of purified horse kidney are comminuted and then homogenized with 5 litres of cold (+4°C) distilled water. The homogenizate is stirred for one hour on an ice water bath and then centrifuged. The supernatant is heated to 70°C, maintained at this temperature for 10 minutes, then cooled in an ice water bath and centrifuged. 1,200 g of ammonium sulphate are added to the supernatant, the mixture is allowed to stand overnight, and the resulting suspension is centrifuged. The precipitate is dissolved in a small amount of cold (+4°C) distilled water, and the solution is dialyzed salt-free against distilled water. The dialyzed solution is filtered, and the filtrate is freeze-dried. 3.8 g of aminoacylase are obtained. Activity: hydrolysis of 319 $\mu$moles of N-acetyl-L-methionine/hour/mg of protein.

Example 6

3 g of pig kidney aminoacylase, prepared as described in Example 3, are dissolved in a 0.05 molar potassium phosphate buffer (pH=7.0), and 50 g of a DEAE Sephadex A-50® resin, previously equilibrated with the same buffer, are added to the solution. The resulting suspension is stirred on an ice water bath for 3 hours and then alllowed to stand overnight in a refrigerator at +4°C. Next day the resin is washed thrice with 1 litre portions of the above buffer, then it is taken up in 1 litre of the same buffer, and the suspension is filled into a column 5.3 cm in diameter and 55 cm in height. The resin column is eluted with a 0.05 molar potassium phosphate buffer (pH=7.0) also containing 0.3 moles of sodium chloride, at a flow rate of 200 ml/hour. The effluent is collected in fractions of 100 ml. The fractions that show enzyme activity are combined, and 80 g of ammonium sulphate are added to the resulting solution by which a suspension of aminoacylase is obtained. 0.7 g of aminoacylase are obtained. Activity: hydrolysis of 5,240 $\mu$moles of N-acetyl-L-methionine/hour/mg of protein.

Example 7

25 ml of the suspension of aminoacylase prepared as described in Example 6, which contains 57 mg of protein, are centrifuged, and the precipitate is dissolved in 9 ml of a 0.05 molar potassium phosphate buffer (pH=7.0). The solution is applied onto the top of a column, 2,4 cm in diameter and 43 cm in height, filled with Sephadex G-200® gel previously equilibrated with the same buffer. The gel column is eluted with the same buffer at a flow rate of 60 ml/hour. The effluent is collected in fractions of 5 ml. The fractions that show enzyme activity are combined, and 9 g of ammonium sulphate are added to the solution by which a suspension of aminoacylase is obtained. 25 mg of aminoacylase are obtained. Activity: hydrolysis of 10,000 $\mu$moles of N-acetyl-L-methionine/hour/mg of protein.

**Claims**

1. A process for isolating aminoacylase enzyme from a mammal kidney extract by means of a heat treatment, precipitating treatments with salts of the general formula $K_xA$ wherein K represents a monovalent cation, A is the anion of a mineral acid and x is equal to the valence of the anion, separating and dissolving precipitates in water and dialysis as well as optionally 1 or more purifying steps, characterized in that as a mammal kidney extract an aqueous kidney extract, prepared in a manner known per se, is subjected to heat treatment at 60 to 80°C for 5 to 15 minutes, the heat

treated mixture is centrifuged, 200 to 300 g/litre of a salt of the general formula K$_x$A are added to the supernatant liquid, the resulting suspension is centrifuged, the separated precipitate is dissolved in water and the solution is dialysed against water and either

a) the enzyme-containing solid is separated from the dialysed solution in a manner known per se, or

b) $\alpha$) the dialysed solution or a solution of the enzyme separated from the dialysate in a manner known per se in a buffer of pH 6.5 to 8.5 is subjected to anion exchange chromatography, and if desired,

$\beta$) the active fractions obtained in the chromatographic step or a solution of the enzyme precipitated from the active fractions with a salt of the general formula K$_x$A in a buffer of pH 6.0 to 8.0 is subjected to gel chromatography on a gel filter wherein the upper limit of the fractionation molecular weight is higher than 70,000

and the enzyme is precipitated from the active fractions [obtained according to $\alpha$) or $\alpha$) and $\beta$)] with a salt of the general formula K$_x$A as described above.

2. A process as claimed in claim 1, characterized in that heat treatment is performed at 70°C for 10 minutes.

3. A process as claimed in claim 1 or 2, characterized in that ammonium sulphate is applied as a salt of the general formula K$_x$A.

**Patentansprüche**

1. Ein Verfahren zum Isolieren des Aminoacylaseenzymes aus einem Säugetiernierenextrakt mittels einer Wärmebehandlung, Fällungsbehandlungen mit Salzen der allgemeinen Formel K$_x$A, worin K ein einwertiges Kation darstellt, A das Anion einer Mineralsäure ist und x der Valenz des Aniones gleich ist, des Trennens und Lösens von Niederschlägen in Wasser und Dialyse sowie gegebenenfalls 1 oder mehr Reinigungsstufen, dadurch gekennzeichnet, daß als Säugetiernierenextrakt ein in an sich bekannter Weise hergestellter wäßriger Nierenextrakt einer Wärmebehandlung bei 60 bis 80°C während 5 bis 15 Minuten unterworfen wird, die wärmebehandelte Mischung zentrifugiert wird, 200 bis 300 g/Liter eines Salzes der allgemeinen Formel K$_x$A zur überstehenden Flüssigkeit zugegeben werden, die erhaltene Suspension zentrifugiert wird, der abgetrennte Niederschlag in Wasser gelöst wird und die Lösung gegen Wasser dialysiert wird und, entweder

a) der enzymhaltige Feststoff von der dialysierten Lösung in an sich bekannter Weise getrennt wird, oder

b) $\alpha$) die dialysierte Lösung oder eine Lösung des vom Dialysat in an sich bekannter Weise getrennten Enzymes in einem Puffer mit einem pH-Wert von 6,5 bis 8,5 der Anionenaustauschchromatographie unterworfen wird und, falls es erwünscht ist,

$\beta$) die in der Chromatographierstufe erhaltenen aktiven Fraktionen oder eine Lösung des von der aktiven Fraktionen gefällten Enzymes mit einem Salz der allgemeinen Formel K$_x$A in einem Puffer mit einem pH-Wert von 6,0 bis 8,0 der Gelchromatographie auf einem Gelfilter, worin die obere Grenze des Fraktioniermolekulargewichtes höher als 70 000 ist, unterworfen wird

und das Enzym von den [gemäß $\alpha$) oder $\alpha$) und $\beta$) erhaltenen] aktiven Fraktionen mit einem Salz der allgemeinen Formel K$_x$A wie oben beschrieben gefällt wird.

2. Ein Verfahren nach Anspruch 1, dadurch gekennzeichnet, daß die Wärmebehandlung 10 Minuten lang bei 70°C durch-geführt wird.

3. Ein Verfahren nach Anspruch 1 oder 2, dadurch gekennzeichnet daß als Salz der allgemeinen Formel K$_x$A Ammoniumsulfat verwendet wird.

**Revendications**

1. Procédé d'isolation d'amino-acylase à partir d'un extrait de reins de mammifères au moyen d'un traitement thermique, de traitements de précipitation avec des sels de formule générale K$_x$A, dans laquelle K représente un cation monovalent, A est l'anion d'un acide minéral et x est égal à la valence de l'anion, de séparation et de dissolution des précipités dans l'eau et de dialyse, ainsi qu'éventuellement d'une ou plusieurs étapes de purification, caractérisé en ce qu'en tant qu'extrait de reins de mammifères, un extrait aqueux de reins, préparé selon une méthode connue en elle-même, est soumis à un traitement thermique à 60—80°C pendant 5 à 15 minutes, le mélange traité par la chaleur est centrifugé, 200 à 300 g/litre d'un sel de formule générale K$_x$A sont ajoutés au liquide surnageant, la suspension résultante est centrifugée, le précipité séparé est dissous dans l'eau et la solution est dialysée vis-à-vis d'eau et, soit

a) le solide contenant l'enzyme est séparé, selon une méthode connue en elle-même, de la solution dialysée, soit

b) $\alpha$) la solution dialysée ou une solution de l'enzyme séparé à partir du dialysat selon une méthode connue en elle-même, dans un tampon pH de 6,5 à 8,5, est soumise à une chromatographie avec échange d'anion et, si cela est désiré,

$\beta$) les fractions actives obtenues dans l'étape chromatographique ou une solution de l'enzyme précipité à partir des fractions actives avec un sel de formule générale $K_xA$ dans un tampon de pH 6,0 à 8,0, sont soumises à une chromatographie sur gel sur un filtre de gel dans lequel la limite supérieure du poids moléculaire de fractionnement est supérieure à 70 000,

et l'enzyme est précipitée à partir des fractions actives [obtenues conformément à $\alpha$) ou $\alpha$) et $\beta$)] avec un sel de formule générale $K_xA$ tel qu'il est décrit plus haut.

2. Procédé selon la revendication 1, caractérisé en ce que le traitement thermique est réalisé à 70°C pendant 10 minutes.

3. Procédé selon l'une quelconque des revendications 1 ou 2, caractérisé en ce que du sulfate d'ammonium est appliqué en tant que sel de formule générale $K_xA$.